# FASCICULE DE BREVET EUROPEEN

(11) **EP 0 326 869 B1**
(45) Date de publication et mention de la délivrance du brevet: **18.08.1993**
(21) Numéro de dépôt: 89100930.0
(22) Date de dépôt: 20.01.1989
(51) Int. Cl.: C07C 403/16, C11B 9/00, A61K 7/46

(54) **Cétones cycloaliphatiques, méthode pour leur préparation et leur utilisation à titre d'ingrédients parfumants et aromatisants**
Cycloaliphatische Ketone, Verfahren zu deren Herstellung und ihre Verwendung als Parfümierungs- und Aromatisierungsmittel
Cycloaliphatic ketones, process for their preparation and their use as perfuming and aromatising ingredients

(30) Priorité: 05.02.1988 CH 418/88; 15.09.1988 CH 3441/88
(43) Date de publication de la demande: 09.08.1989
(73) Titulaire: FIRMENICH SA, CH-1211 Genève 8 (CH)
(72) Inventeur: Fehr, Charles, CH-1290 Versoix (CH); Galindo, José, CH-1220 Les Avanchets (CH)
(74) Mandataire: Salvadori, Giuseppe

(56) Documents cités:
- US-A- 4 172 850
- J. AM. CHEM. SOC., vol. 110, septembre 1988, pages 6911-6913; C. FEHR et al.: "Synthesis of (R)-(+)- and (S)-(-)-alpha-damascone by tandem grignard reaction-enantioselective protonation: Evidence for the intermediacy of a chiral complex"
- CHEM. PHARM. BULL., vol. 23, no. 2, 1975, pages 279-284; M. SHIBASAKI et al.: "Stereochemical studies. XXXV. A biogenetic-type asymmetric cyclization. Syntheses of optically active alpha-cyclocitral and trans-alpha-damascone"
- TETRAHEDRON, vol. 42, no. 12, 1986, pages 3245-3250; F. NAEF et al.: "Grignard and hydride addition to a ketene intermediate: A novel access to alpha-damascone and alpha-cyclocitral"

## Description

La présente invention a trait au domaine de la parfumerie et des arômes. Elle concerne plus particulièrement des isomères optiquement actifs de l'alpha-damascone, notamment l'énantiomère de structure 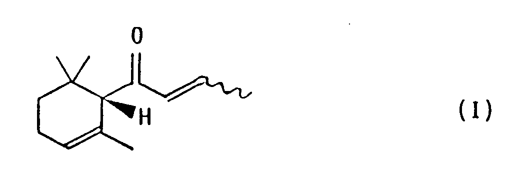
ou (-)-(1′S)-1-(2′,2′,6′-triméthyl-2′-cyclohexène-1′-yl)-2-butène-1-one ainsi que son isomère -3-butène-1-one correspondant.

Depuis leur découverte [voir brevets suisses N° 509,399 et 524,320 ; Helv. Chim. Acta, 53, 541(1970)], l'intérêt porté aux damascones et ses dérivés n'a cessé de croître auprès des parfumeurs et aromaticiens. Leurs qualités organoleptiques en font des ingrédients de choix dans grand nombre de compositions à usage varié. Leur domaine d'application s'étend tant à la parfumerie de luxe qu'au parfumage de produits et articles techniques, tels les savons et les détergents.

L'alpha-damascone, en particulier, a trouvé une utilisation étendue dans des compositions à caractère fruité et floral et ses caractères de pomme verte ont permis la création de notes olfactives inédites fort appréciées. Nombreuses sont les publications relatives à des procédés pour sa préparation. Dans la plupart des cas examinés, elles se réfèrent à la préparation du composé racémique.

Préparée à l'aide de différents procédés de synthèse, l'alpha-damascone est utilisée en effet sous forme d'un mélange racémique.

G. Ohloff et G. Uhde [Helv. Chim. Acta, 53, 531(1970)] ont toutefois décrit un procédé pour la préparation de (R)-(+)-alpha-damascone, ou (+)-(1′R,E)- et (+)-(1′R,Z)-1-(2′,2′,6′-triméthyl-2′-cyclohexène-1′-yl)-2-butène-1-one de formule 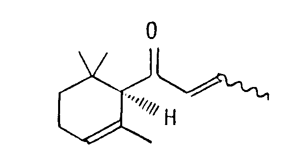
Le procédé est caractérisé par une oxydation au bioxyde de manganèse de l'alcool secondaire correspondant lequel est obtenu à partir de (+)-alpha-ionone suivant le schéma réactionnel que voici : 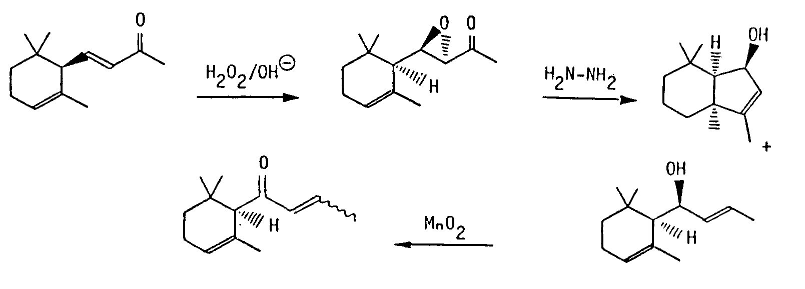
Le composé ainsi obtenu a montré un [alpha]²⁰_{D} = + 324° pour la forme E, et un [alpha]²⁰_{D} = + 340°pour l'isomère Z.

M. Shibasaki et al. [Chem. Pharm. Bull. 23,279(1975)] ont également décrit un procédé de synthèse de ce même énantiomère de l'alpha-damascone, lequel procédé est caractérisé par une cyclisation diastéréomérique du citral à l'aide d'un auxiliaire chiral à travers la formation de ses énamines, comme indiqué ci-après : 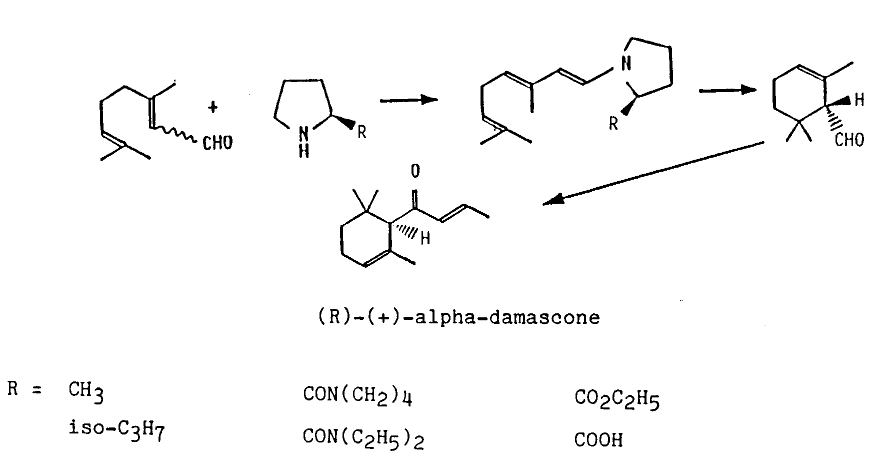
En se référant aux valeurs fournies par Ohloff et Uhde, le composé ainsi obtenu a été défini par les auteurs cités comme possédant une pureté optique de 27,5% et un [alpha]²⁰_{D} = + 89,2°.

Nous avons pu établir que l'isomère (R)-(+) ainsi préparé, tout en étant caractérisé par une note olfactive dominée par un accord fruité et floral plaisant, possédait également un aspect qui pouvait rappeler une odeur de type "bouchon" qui rendait son utilisation problèmatique dans bien des applications.

Cette observation nous a incités à examiner de près quelles pouvaient être les caractéristiques organoleptiques de l'autre énantiomère, la (S)-(-)-alpha-damascone. Toutefois, comme l'état de la technique ne nous offrait pas le moyen d'accéder à ce produit par manque d'une synthèse appropriée, nous avons développé un procédé énantiosélectif original. Ce procédé constitue également l'un des objets de la présente invention.

Ledit procédé de l'invention est caractérisé en ce qu'on traite un énolate de formule 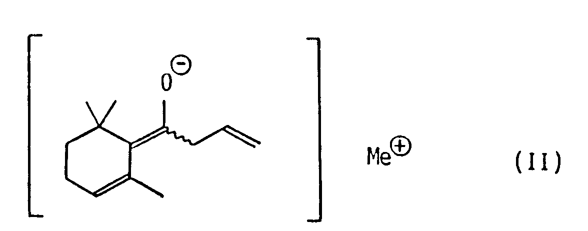
dans laquelle Me définit un atome d'un métal alcalin de préférence le lithium, ou le magnésium, avec un réactif chiral donneur de protons constitué par un dérivé azoté bifonctionnel de formule 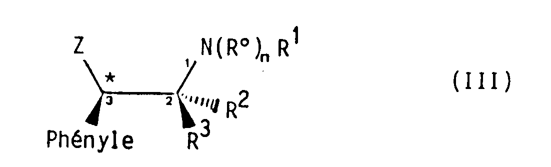
dans laquelle
l'indice n vaut zéro ou 1;
les symboles R° et R¹ définissent chacun un radical alkyle linéaire ou ramifié, ou l'un des deux représente un atome d'hydrogène et l'autre un radical alkyle tel que défini plus haut ;
R² et R³ définissent chacun un radical alkyle linéaire ou ramifié, ou l'un des deux représente un atome d'hydrogène et l'autre un radical alkyle tel que défini plus haut ;
. et Z sert à définir un groupe OH ou un radical bivalent HN-C(O), dont l'atome d'azote est relié à l'atome de carbone en position 3 et le groupe carbonyle est relié à l'atome d'azote en position 1
. et dans laquelle l'atome d'azote en position 1 peut être relié à un groupe benzylique d'une résine polystyrénique ;
hydrolyse ensuite le mélange réactionnel et isomérise au moyen d'un agent isomérisant selon les méthodes usuelles. [L'astérisque⁽*⁾ indique un centre de chiralité].

En tant que réactif chiral donneur de protons on peut utiliser à titre préférentiel une hydroxy-amine de formule 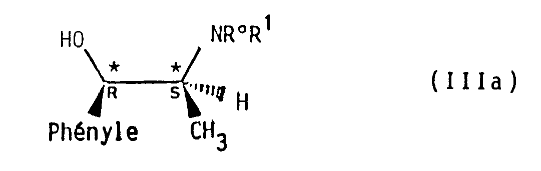
par exemple le (1R,2S)-2-(méthylamino)-1-phénylpropan-1-ol, ou 1-éphédrine, le (1R,2S)-2-(diméthylamino)-1-phénylpropan-1-ol, le (1R,2S)-2-(isopropylamino)-1-phénylpropan-1-ol, le (1R,2S)-2-(N-méthyl-N-isopropylamino)-1-phénylpropan-1-ol, ou un dérivé cyclique de l'urée de formule 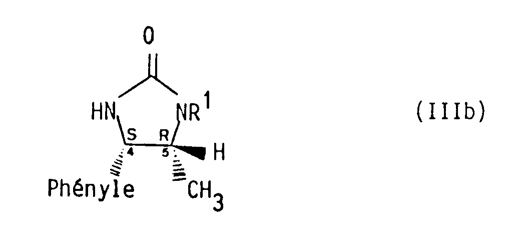
par exemple les composés pour lesquels R¹ = CH₃ ou isopropyle.

La première classe des réactifs mentionnés ci-dessus appartient à la catégorie des dérivés de l'éphédrine, lesquels peuvent être obtenus soit à partir de l'éphédrine elle-même, soit à partir d'acide mandélique, produits de départ économiques aisément disponibles sur le marché.

Le deuxième type de composés appartient à la classe des urées cycliques dont la préparation est décrite dans l'art antérieur.

C'est ainsi que le 1-2-(N-méthyl-N-isopropylamino)-1-phénylpropan-1-ol peut être obtenu à partir de 1-éphédrine par condensation au moyen d'acétone dans l'éthanol, suivie de réduction au moyen de NaBH₄, tandis que le 1-2-(isopropylamino)-1-phénylpropan-1-ol est obtenu par une voie analogue à partir de 1-noréphédrine suivant J. E. Saavedra [J. Org. Chem., 50, 2271 (1985)].

Les autres réactifs chiraux donneurs de protons à structure analogue peuvent être préparés par des méthodes similaires.

Appartiennent à cette classe de composés chiraux également les dérivés polymériques du type décrit dans la littérature [voir J. M. J. Fréchet et al., J. Org. Chem., 51, 3462 (1986)]. Il s'agit de résines polystyréniques dans lesquelles l'atome d'azote en position 1 des composés (III) est relié au groupe p-méthylène des restes aromatiques de la résine.

Il s'agit par exemple des résines de formule 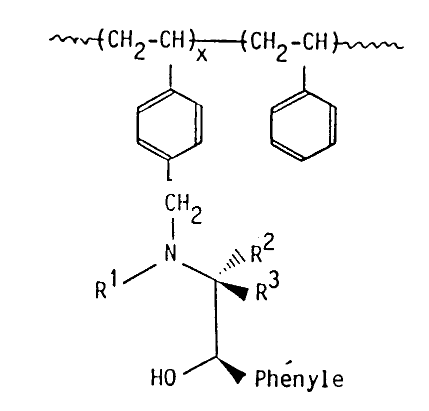
Ces composés présentent l'avantage considérable de pouvoir être régénérés facilement par filtration.

En ce qui concerne l'autre classe de réactifs, dérivés cycliques de l'urée, ils peuvent également être obtenus par des procédés connus. C'est ainsi que la (+)-(4S,5R)-1,5-diméthyl-4-phényl-2-imidazolidone peut être obtenue à partir de chlorhydrate de (+)-éphédrine par réaction avec de l'urée ( [alpha]²⁰_{D} = +44,5° (c=3; CH₃OH)) suivant H. Roder et al., Angew., Chem., 96, 895 (1984).

L'hydrolyse du mélange réactionnel obtenu peut s'effectuer au moyen d'une solution aqueuse acide, par exemple à l'aide d'un acide protique dilué, tel l'acide chlorhydrique ou sulfurique ou par traitement avec une solution aqueuse de chlorure d'ammonium, de préférence à une température inférieure à la température ambiante. L'étape finale du procédé de l'invention, qui consiste en l'isomérisation de la double liaison terminale du composé obtenu par hydrolyse, s'effectue conformément à des méthodes en soi connues, par exemple par traitement dudit composé au moyen d'un agent d'isomérisation acide suivant la méthode décrite dans le brevet suisse n° 537.352 ou par traitement avec de l'alumine.

L'énolate de formule (II) utilisé comme produit de départ dans le procédé décrit plus haut peut être obtenu aisément par traitement d'un ester de l'acide cyclogéranique de formule 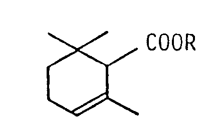
dans laquelle le symbole R sert à définir un reste alkyle linéaire inférieur, le méthyle ou l'éthyle de préférence, au moyen d'une base forte telle un alkyl ou le phényl-lithium. A cet effet, le n-butyl-lithium est utilisé de préférence.

La présente invention a également trait à un procédé original pour la préparation de la (S)-(-)-alpha-damascone de formule (I), lequel procédé est caractérisé en ce qu'on
a. traite un composé organo-magnésien de formule 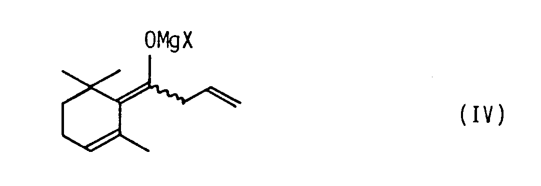 dans laquelle la ligne ondulée a la sens indiqué plus haut et X désigne un atome d'halogène avec un équivalent au moins d'un alcoolate de lithium ;
b. additionne au mélange réactionnel un équivalent au moins d'un réactif chiral donneur de protons constitué par un dérivé azoté bifonctionnel de formule (III) ;
c. hydrolyse ensuite le mélange réactionnel et isomérise au moyen d'un agent isomérisant selon les méthodes usuelles.

A titre d'alcoolate de lithium, on peut utiliser un alcoolate dérivé d'un alcool inférieur tel le méthanol, l'éthanol, l'isopropanol ou le butanol, de préférence le tert-butanol. Des sels lithiés dérivés d'anions chiraux peuvent également être utilisés à cet effet. On choisit dans ce cas des dérivés lithiés des composés azotés hydroxylés de formule (III) dans laquelle Z sert à définir un groupe OH, en particulier on utilise le (1R,2S)-2-(N-méthyl-N-isopropylamino)-1-phénylpropanolate de lithium.

A titre de réactif chiral donneur de protons, on utilise un dérivé azoté bifonctionnel de formule (III) tel que défini plus haut.

Lorsque la première étape du procédé est effectuée au moyen d'un sel de lithium dérivé d'un anion chiral, l'étape successive peut être effectuée en ayant recours à un donneur de protons achiral, tel un alcool aliphatique comme le tert-butanol.

Une variante du procédé décrit consiste donc à
a′. traiter un composé organo-magnésien de formule (IV) avec un équivalent au moins d'un alcoolate de lithium constitué par un sel lithié d'un composé azoté hydroxylé de formule (III) dans laquelle Z sert à définir un groupe OH ; et
b′. additionner au mélange réactionnel un équivalent au moins d'un donneur de protons constitué par un alcool aliphatique, en particulier le tert-butanol.

Les étapes successives d'hydrolyse et d'isomérisation se feront dans ce cas comme décrit précédemment.

Les composés organo-magnésiens de formule (IV) utilisés comme produits de départ dans le procédé décrit ci-dessus peuvent être obtenus à partir du cétène correspondant ou 2,6,6-triméthyl-cyclohex-2-énylcétène, lui-même préparé suivant un procédé connu [voir J. Org. Chem., 42, 2111 (1977)].

Sans vouloir être limités par des considérations ayant trait au mécanisme des réactions qui caractérisent le procédé décrit ci-dessus, nous nous sommes forgés l'opinion, comme suite aux observations accumulées lors des différents essais effectués dans la mise en oeuvre du procédé, que la formation d'un complexe mixte 1:1 lithium-magnésium entre l'organo-magnésien et l'alcoolate de lithium représente une étape critique pour l'obtention d'un degré élevé d'énantiosélectivité.

C'est de façon inattendue que nous avons constaté que les propriétés organoleptiques de l'énantiomère (S)-(-) de l'alpha-damascone étaient caractérisées par une note florale plus marquée et plus fraîche que celle du composé racémique connu. Son caractère olfactif rappelait les pétales de rose. La (S)-(-)-alpha-damascone présentait en outre un caractère vert et légèrement vineux sans posséder la connotation "bouchon" et la note pomme verte typique du mélange racémique ou de l'énantiomère (R)-(+).

Vis-à-vis de ce dernier, la (S)-(-)-alpha-damascone présente des différences marquées non seulement du point de vue qualitatif, mais également en ce qui concerne sa puissance et son intensité, celle de la (S)-(-)-alpha-damascone étant nettement supérieure à celle de l'énantiomère (R)-(+) correspondant. Une évaluation de leur seuil de perception respectif a montré que la (S)-(-)-alpha-damascone, avec une valeur de 1,5 ppb (parties par milliard), était environ 65 fois plus puissante que la (R)-(+)-alpha-damascone dont le seuil de perception était de 100 ppb.

Bien entendu, ce fait se traduit dans la pratique par une économie accrue pour l'utilisateur, le nouveau composé pouvant offrir une puissance odorante et aromatisante supérieure à celle observée par l'emploi de quantités équivalentes de l'énantiomère (R)-(+) connu.

Lorsque la (S)-(-)-alpha-damascone est employée à titre d'ingrédient parfumant, elle peut servir au parfumage de produits aussi variés que des savons, des détergents liquides ou solides, des adoucissants textiles ou des produits d'entretien par exemple. Elle peut également servir à la préparation de compositions parfumantes destinées à la parfumerie fine et être utilisée soit en l'état, soit en mélange avec d'autres ingrédients d'origine naturelle ou de synthèse. A cet effet les ingrédients parfumants usuels peuvent être employés pour autant que les conditions d'équilibre et d'harmonie dans la composition spécifique considérée soient respectées.

Les concentrations efficaces de (S)-(-)-alpha-damascone dans les applications de parfumerie varient, comme souvent en pareil cas, dans des limites assez larges. Lors du parfumage de produits tels que savons ou détergents par exemple, ces valeurs de concentrations peuvent être de l'ordre de 0,1-0,5% en poids par rapport au poids du produit fini. Ces valeurs peuvent être augmentées à 1-5%, voire plus, lors de la préparation de compositions parfumantes ou de concentrés.

Dans des applications arômes la (S)-(-)-alpha-damascone est caractérisée par une note florale. Elle rappelle en outre le thé par son caractère herbal. La (R)-(+)-alpha-damascone présente par contre un caractère boisé, camphré, moisi et sale.

Il est intéressant de remarquer que la (RS)-alpha-damascone possède une note aromatique fruitée plus prononcée que l'énantiomère (S)-(-). Au vu de ses caractéristiques organoleptiques, la (S)-(-)-alpha-damascone convient tout particulièrement à conférer, modifier ou améliorer les propriétés aromatiques d'aliments et boissons de nature variée. Elle peut servir par exemple à aromatiser des infusions ou décoctions, des produits de pâtisserie ou boulangerie, des confitures, voire le tabac.

Les proportions dans lesquelles la (S)-(-)-alpha-damascone sert à obtenir les effets aromatisants désirés varient dans une gamme de valeurs assez étendue. L'homme de l'art sait par expérience que la valeur de ces concentrations peut dépendre de l'effet particulier souhaité, de la nature du produit à aromatiser ainsi que de celle des autres ingrédients aromatisants présents dans une composition donnée.

A titre de coingrédients aromatisants, on peut utiliser les composés connus dans l'art et amplement décrits dans la littérature technique [voir par exemple S. Arctander, Perfume and Flavor Chemicals, Montclair, N. J.,(1969) ; Fenaroli's Handbook of Flavor Ingredients, 2nd Edition, CRC Press, Inc. (1975)]. Il en va de même pour les diluants ou les supports. A cet effet on peut employer l'éthanol, la triacétine ou le dipropylène glycol ou des supports solides tels la gomme arabique ou les dextrines.

La présente invention est illustrée d'une manière plus détaillée par les exemples suivants dans lesquels les températures sont indiquées en degrés centigrades et les abréviations ont le sens usuel dans l'art.

### Exemple 1

### Procédé de préparation de la (-)-(1′S,E)-1-(2′,2′,6′-triméthyl-2′-cyclohexène-1′-yl)-2-butène-1-one

a. 150,6 G (0,83M) d'alpha-cyclogéraniate de méthyle dans 1,5 l de tétrahydrofuranne (THF) anhydre ont été traités à -10° avec 1,2 équivalents de butyl-lithium. Après avoir laissé monter la température à environ 15°, on a ajouté au mélange réactionnel 1,35 équivalents de chlorure d'allyl-magnésium dans le THF et laissé réagir à 35° pendant 30 mn.
b. Le mélange a été ensuite refroidi de nouveau à -10° et à cette température on y a ajouté 237g (1,5 équivalents) de (+)-(4S,5R)-1,5-diméthyl-4-phényl-2-imidazolidone en 1 minute et on a laissé réagir pendant 30 minutes à environ -10°/0°, puis le mélange réactionnel a été versé sur un mélange NH₄Cl-glace. La phase organique, une fois séparée, a été lavée à l'eau et une solution aqueuse saturée de chlorure de sodium, séchée sur du Na₂SO₄ anhydre, filtrée et concentrée. Le résidu a été repris avec de l'éther de pétrole 30°/50° et filtré.
   Le filtrat clair a été évaporé et distillé sur résidu et une fraction constituée par de la (-)-(1′S)-1-(2′,2′,6′-triméthyl-2′-cyclohexène-1′- yl)-3-butène-1-one ayant eb. 50°-70°/10,6 Pa a été ainsi recueillie ; [alpha]²⁰_{D}(liquide) = -260° ± 20°.
   Cette dernière a été soumise à une distillation fractionnée, (eb. 50-55°/10.6 Pa) traitée avec de l'alumine selon Reetz et al. [Chem. Ber., 118, 348(1985)] et redistillée sur résidu pour fournir une fraction de 89 g de la cétone désirée ayant une pureté de 97%, telle qu'indiquée par analyse gas-chromatographique ([alpha]²⁰_{D} = -280° ± 20° dans le CHCl₃). Par cristallisations successives au pentane (5x) du produit obtenu on a pu isoler la (-)-(1′S,E)-1-(2′,2′,6′-triméthyl-2′-cyclohexène-1′-yl)-2-butène-1-one optiquement pure ayant [alpha]²⁰_{D} = - 509,7° (c=4,0 dans le CHCl₃); F = env. 27°.

En effectuant la réaction comme indiqué ci-dessus mais en utilisant le (1R,2S)-2-(N-méthyl-N-isopropylamino)-1-phénylpropan-1-ol à la place de (+)-(4R,5S)-1,5-diméthyl-4-phényl-2-imidazolidone on obtient le composé désiré qui, avant cristallisation, montrait un [alpha]²⁰_{D} = - 340° ± 20° (c=6,4 ; CHCl₃).

### Exemple 2

### Procédé de préparation de la (-)-(1′S,E)-1-(2′,2′,6′-triméthyl-2′-cyclohexène-1′-yl)-2-butène-1-one

10 g (66,6 mMol) de 2,6,6-triméthyl-cyclohex-2-énylcétène [voir J. Org. Chem., 42, 2111 (1977)] dans 200 ml de tétrahydrofuranne (THF) anhydre ont été traités successivement avec 1,2 équivalents de chlorure d'allyl-magnésium dans du THF [temp. : -78°→35° ; temps : 30 mn], puis à 20° pendant 30 mn avec un équivalent de (1R,2S)-2-(N-méthyl-N-isopropylamino)-1-phénylpropanolate de lithium [obtenu par traitement d'un équivalent de (1R,2S)-2-(N-méthyl-N-isopropylamino)-1-phénylpropanol avec un équivalent de n-butyl-lithium dans le THF], et enfin avec 2 équivalents de (1R,2S)-2-(N-méthyl-N-isopropylamino-)-1-phénylpropanol. L'addition de ce dernier réactif s'effectue à une température comprise entre -50° et -10° pendant 60 mn.
Le mélange de réaction a été versé dans une solution glacée aqueuse de NH₄Cl et extrait à l'éther. Les phases organiques combinées ont été traitées avec une solution aqueuse à 5% d'HCl, puis les phases aqueuses séparées ont été lavées à l'éther, traitées avec une solution aqueuse de KOH à 20% et extraites à l'éther pour fournir une solution éthérée qui, après évaporation, a donné du (1R,2S)-2-(N-méthyl-N-isopropylamino)-1-phénylpropanol.
Le mélange de cétones obtenu des extraits organiques a été distillé dans un four à boules à 70°, sous 0,5 Torr, pour fournir 9,7 g d'un produit qui, par isomérisation avec de l'alumine selon Reetz et al. [Chem. Ber., 118, 348 (1985)], a donné 9,3 g (73%) de (-)-(1′S,E)-1-(2′,2′,6′-triméthyl-2′-cyclohexène-1′-yl)-2-butène-1-one.
[alpha]²⁰_{D} = -396° (c = 4,0 ; CHCl₃)
Par cristallisations successives au pentane du produit obtenu, on a pu isoler la cétone désirée ayant un [alpha]²⁰_{D} = -488° (c = 4,0 ; CHCl₃) ; F. = 27,5-28°.

### Exemple 3

### Mesure du seuil de perception

Deux échantillons, respectivement de (-)-(1′S,E)-1-(2′,2′,6′-triméthyl-2′-cyclohexène-1′-yl)-2-butène-1-one (ou (S)-(-)-alpha-damascone) et de (R)-(+)-alpha-damascone ont été soumis à l'évaluation d'un groupe d'experts composé par 17 à 20 individus.

Les deux échantillons ont été évalués en ayant recours à la méthode de Guadagni [voir Guadagni et al., J. Sci. Food Agric., 14, 761(1963)].

Les concentrations des échantillons ont été arrangées en ordre décroissant de sorte à diminuer les erreurs éventuelles dues à la fatigue. Les produits ont été évalués en les dissolvant dans de l'eau minérale naturelle. Chaque produit est goûté à différents dosages par comparaison avec un échantillon d'eau.

Trois séances d'évaluation ont été effectuées dans un intervalle d'une semaine afin de vérifier la reproductibilité des résultats obtenus. 70% de réponses correctes ont été considérées comme définissant la valeur du seuil de perception.

Les valeurs observées ont été les suivantes :
(S)-(-)-alpha-damascone : 1,5 ppb (parties par milliard)
(R)-(+)-alpha-damascone : 100 ppb

### Exemple 4

Une composition parfumante de base de type florale a été préparée en mélangeant les ingrédients suivants (parties en poids) :

| | |
|---|---|
| Citronellol | 150 |
| Phényléthanol | 150 |
| Terpinéol | 70 |
| Lilial (marque enregistrée)¹⁾ | 50 |
| Salicylate de benzyle | 100 |
| Trichloro-méthyl-phényl carbinyl acétate | 30 |
| Ald. cyclamen | 10 |
| Ald. undécylénique 10%* | 20 |
| Oxyde de rose ²⁾ 10%* | 20 |
| Indol purifié 10%* | 10 |
| Acétate de styrallyle | 10 |
| Linalol | 80 |
| Total | 7̅0̅0̅ |

| | |
|---|---|
| 1) L. Givaudan ; ald. p-tert-butyl-alpha-méthyl hydrocinnamique | |
| 2) Firmenich SA | |
| *) dans le dipropylène-glycol | |

A l'aide de la base parfumante ainsi obtenue on a préparé quatre nouvelles compositions en mélangeant les ingrédients suivants :

| | A | B | C | D |
|---|---|---|---|---|
| Base parfumante | 70 | 70 | 70 | 70 |
| Dipropylène-glycol | 30 | - | - | - |
| (RS)-alpha-damascone* | - | 30 | - | - |
| (S)-(-)-alpha-damascone* | - | - | 30 | - |
| (R)-(+)-alpha-damascone* | - | - | - | 30 |

| | | | | |
|---|---|---|---|---|
| * solution à 10% dans le dipropylène-glycol | | | | |

Ces nouvelles compositions ont été soumises à une évaluation olfactive de la part d'un groupe d'experts.

Leurs commentaires ont été les suivants :
Composition
A : caractère plat, sans odeur rosée définie ;
B : odeur rosée plaisante, note légèrement "bouchon", vineuse ;
C : odeur fraîche, rosée bien marquée direction pétales de rose ; le plus plaisant des quatre échantillons soumis ; sans caractère secondaire gênant, le plus puissant ;
D : échantillon le moins intéressant ; note "bouchon" marquée, caractère chimique.

### Exemple 5

### Evaluation aromatique comparative

Une évaluation comparative entre les deux énantiomères optiquement actifs (R)-(+) et (S)-(-) de l'alpha-damascone a été effectuée par un groupe d'experts composé par 10 individus des deux sexes. Les deux composés ont été goûtés à raison de 1 ppm (partie par million) en solution dans un sirop de sucre préalablement dilué à 10%.

Les deux échantillons ont été jugés comme étant : (S)-(-)-alpha-damascone : boisé, herbal, fruité (direction baies), fruit cuit, tabac, thé, paille, feuilles sèches ; (R)-(+)-alpha-damascone : paille, camphré, huileux, fermenté, noisette, sale, sans volume.

### Exemples 6-10

### Exemple 6

Un jus de pruneau concentré commercial a été dilué avec de l'eau de source et divisé en quatre parties d'égal volume :
A : échantillon non aromatisé de référence ;
B : échantillon auquel on a ajouté de la (RS)-alpha-damascone à une concentration de 0,5 ppm ;
C : échantillon auquel on a ajouté de la (R)-(+)-alpha-damascone à une concentration de 0,5 ppm ;
D : échantillon auquel on a ajouté de la (S)-(-)-alpha-damascone à une concentration de 0,5 ppm.

Les quatres échantillons ainsi obtenus ont été soumis pour évaluation à un groupe d'experts aromaticiens qui ont été appelés à se prononcer sur leurs qualités organoleptiques. Leurs commentaires peuvent se résumer ainsi :
A : caractère typique de pruneau, plutôt plat, note de fruit cuit ;
B : plus fruité, juteux et arrondi, caractère de fermentation ;
C : plus boisé, caractère de fruit sec, carton ;
D : caractère de fruit sec encore plus prononcé, légèrement caramel, plus fruité. Plus distinct que B et C. Plus de caractère que A avec une note vineuse ou rhum.

### Exemple 7

Un jus de framboise concentré du commerce a été dilué avec de l'eau de source et divisé en quatre parties de volume égale. On a procédé comme indiqué à l'exemple précédent en ajoutant à trois des échantillons de la (RS)-alpha-damascone, de la (R)-(+)-alpha-damascone et de la (S)-(-)-alpha damascone respectivement. Les commentaires exprimés par les aromaticiens appelés à évaluer leurs propriétés organoleptiques, par comparaison avec un échantillon de jus non aromatisé, sont résumés ci-après :
A : caractère typique d'un jus de framboise conditionné, note de fruit cuit, plutôt plat ;
B : paille, camphre, possède plus de corps fruité, légèrement terreux ;
C : semblable à B ; légèrement camphré, bonne note fruitée ; le caractère de "confiture" est renforcé, meilleur que A et B ; légèrement paille ;
D : plus fruité, fruit cuit ; plus juteux et confiture, plus typiquement baie, boisé. Très typiquement framboise. Cet échantillon est préféré car sa note se rapproche le plus de la note naturelle de la framboise même.

### Exemple 8

On a préparé 100 ml de sirop de sucre acidulé par dissolution de 8 g de sucre et 0,1g d'acide citrique dans de l'eau de source. La solution ainsi obtenue a été aromatisée à l'aide d'un arôme concentré de fraise à raison de 0,05 % en poids (origine : Firmenich SA), puis elle a été divisée en quatre parties et on a procédé comme indiqué à l'exemple précédent. La proportion des trois composés à l'examen dans la solution aqueuse était de 0,03 ppm. Voici les commentaires exprimés par les aromaticiens faisant partie du groupe d'évaluation :
A : fruité, confiture de fraise, beurre ;
B : possède plus de corps que A ; camphré, paille, fermenté ;
C : plus fruité que A et B, caractère de confiture plus marqué, fruit cuit ;
D : le plus fruité, possède un caractère plus marqué de baie rouge, note plus définie de confiture. D représente l'échantillon préféré.

### Exemple 9

6 g de feuilles de thé de Ceylan ont servi à la préparation d'une infusion par suspension dans 800 ml d'eau bouillante. Comme indiqué aux exemples précédents on a divisé l'infusion obtenue en quatre parties de volume égale. La proportion des trois composés à l'examen était de 0,25 ppm. Par comparaison avec un échantillon de l'infusion non aromatisée les échantillons B, C et D ont été évalués et définis comme :
B : plus fruité, note abricot, plus floral, terreux ; l'odeur a plus d'impact que celui de l'échantillon non aromatisé A ;
C : floral, caractère de feuilles sèches plus marqué, légèrement terreux, noix ; ressemble à l'échantillon A mais possède plus d'arôme et d'impact ;
D : caractère plus marqué de thé aromatique, feuilles sèches, foin, plus fruité et plus doux. L'échantillon D est préféré, il montre le caractère le plus typique du thé noir.

### Exemple 10

Un échantillon constitué par des cigarettes d'une marque de commerce, fabriquées à l'aide de tabac de type "flue-cured", a été employé dans cet exemple.
On a ensuite préparé trois échantillons de cigarettes aromatisées par injection d'une solution alcoolique (éthanol à 95 %) de (RS)-alpha-damascone (B), (R)-(+)-alpha-damascone (C) et (S)-(-)-alpha-damascone (D), respectivement. Le dosage a été établi de sorte à obtenir une proportion des composés à l'examen de 12,5 ppm par rapport au poids du tabac et les échantillons ont été évalués, après conditionnement pendant 48 heures, par comparaison avec un échantillon de cigarettes non aromatisées.
L'évaluation a porté sur l'arôme qui se dégage de cigarettes à l'ouverture de l'emballage les contenant et sur le goût de la fumée pendant la combustion.

### Odeur à l'ouverture de l'emballage :

A : neutre, boisé ;
B : fruité, légèrement caramel, boisé, fermenté, note tabac plus prononcée ;
C : bonne note de tête tabac, fruité, fermentée, légèrement floral ;
D : note de tabac plus riche, plus fruitée, caractère de paille plus marqué.

### Arôme de la fumée :

A : note tabac typique ;
B : caractère plus complet, plus fruité, lourd, arrière-goût sale ;
C : plus léger, plus herbal, paille, plus doux, caractère "flue-cured" plus prononcé ;
D : plus riche, caractère tabac plus prononcé, plus fruité, type fruit sec, que C. Plus doux que A. Possède plus de corps.

## Revendications

1. (-)-(1'S)-1-(2′,2′,6′-triméthyl-2′-cyclohexène-1′-yl)-2-butène-1-one sous forme pratiquement pure.

2. Composé selon la revendication 1 caractérisé par un [alpha]²⁰_{D} = -488° (CHCl₃ ; c=4,0).

3. (-)-(1'S)-1-(2′,2′,6′-triméthyl-2′-cyclohexène-1′-yl)-3-butène-1-one.

4. Procédé pour la préparation de (-)-(1'S)-1-(2′,2′,6′-triméthyl-2′-cyclohexène-1′-yl)-2-butène-1-one de formule 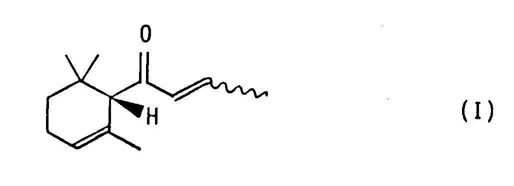 dans laquelle la ligne ondulée désigne une liaison C-C de configuration cis ou trans, caractérisé en ce qu'on traite un énolate de formule 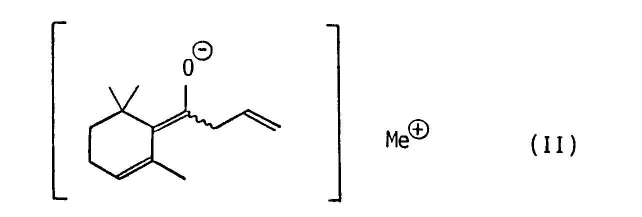 dans laquelle la ligne ondulée a le sens indiqué ci-dessus et Me définit un atome d'un métal alcalin, de préférence le lithium, ou le magnésium, avec un réactif chiral donneur de protons constitué par un dérivé azoté bifonctionnel de formule 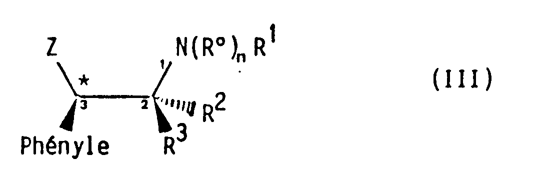 dans laquelle
. l'astérisque définit un centre de chiralité ;
. l'indice n vaut zéro ou 1 ;
. les symboles R° et R¹ définissent chacun un radical alkyle ou aralkyle linéaires ou ramifiés, ou l'un des deux représente un atome d'hydrogène et l'autre un radical alkyle tel que défini plus haut;
. R² et R³ définissent chacun un radical alkyle linéaire ou ramifié, ou l'un des deux représente un atome d'hydrogène et l'autre un radical alkyle tel que défini plus haut; et
. Z sert à définir un groupe OH ou un radical bivalent HN-C(O), dont l'atome d'azote est relié à l'atome de carbone en position 3 et le groupe carbonyle est relié à l'atome d'azote en position 1 ;
. et dans laquelle l'atome d'azote en position 1 peut être relié à un groupe benzylique d'une résine polystyrénique ;
hydrolyse ensuite le mélange réactionnel et isomérise au moyen d'un agent isomérisant selon les méthodes usuelles.

5. Procédé selon la revendication 4, caractérisé en ce qu'on utilise comme réactif chiral donneur de protons de formule (III) une hydroxy-amine de formule 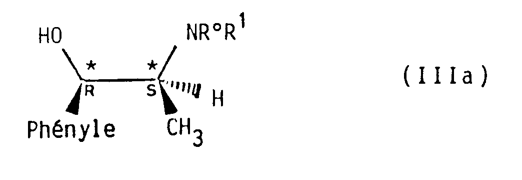 dans laquelle R° et R¹ ont le sens indiqué à la revendication 4.

6. Procédé selon la revendication 5, caractérisé en ce qu'on utilise comme réactif chiral donneur de protons le (1R,2S)-2-(isopropylamino)-1-phénylpropan-1-ol, le (1R,2S)-2-(N-méthyl-N-isopropylamino)-1-phénylpropan-1-ol ou le (1R,2S)-2-(méthylamino)-1-phénylpropan-1-ol.

7. Procédé selon la revendication 5, caractérisé en ce qu'on utilise comme réactif chiral donneur de protons une résine polystyrènique modifiée contenant des unités de (1R,2S)-2-(méthylamino)-1-phénylpropan-1-ol.

8. Procédé selon la revendication 4, caractérisé en ce qu'on utilise comme réactif chiral donneur de protons de formule (III), un dérivé cyclique de l'urée de formule 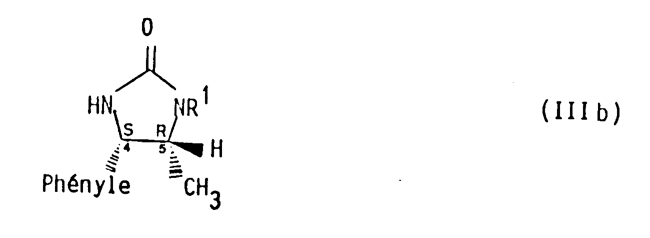 dans laquelle R¹ a le sens indiqué à la revendication 4.

9. Procédé selon la revendication 8, caractérisé en ce qu'on utilise comme réactif chiral donneur de protons le (+)-(4S,5R)-1,5-diméthyl-4-phényl-2-imidazolidone.

10. Procédé pour la préparation de (-)-(1'S)-1-(2′,2′,6′-triméthyl-2′-cyclohexène-1′-yl)-2-butène-1-one de formule 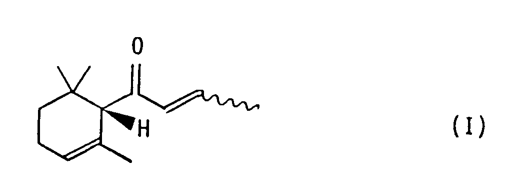 dans laquelle la ligne ondulée désigne une liaison C-C de configuration cis ou trans, caractérisé en ce qu'on
a. traite un composé organo-magnésien de formule 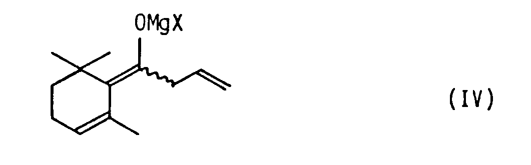 dans laquelle la ligne ondulée a le sens indiqué ci-dessus et X désigne un atome d'halogène, avec un équivalent au moins d'un alcoolate de lithium ;
b. additionne au mélange réactionnel un équivalent au moins d'un réactif chiral donneur de protons constitué par un dérivé azoté bifonctionnel de formule (III) telle que définie à la revendication 4 ;
c. hydrolyse ensuite le mélange réactionnel et isomérise au moyen d'un agent isomérisant selon les méthodes usuelles ; ou
a′. traite un composé organo-magnésien de formule (IV) avec un équivalent au moins d'un alcoolate de lithium constitué par un sel lithié d'un composé azoté hydroxylé de formule (III) telle que définie à la revendication 4 ;
b′. additionne au mélange réactionnel un équivalent au moins d'un donneur de protons constitué par un alcool aliphatique, en particulier le tert-butanol ; et
c′. hydrolyse et isomérise comme indiqué sous lettre c. ci-dessus.

11. Procédé selon la revendication 10 caractérisé en ce qu'on utilise, comme alcoolate de lithium, un sel lithié dérivé d'un alcool inférieur choisi parmi le groupe du méthanol, éthanol, isopropanol et butanol, de préférence tert-butanol.

12. Procédé selon la revendication 10 caractérisé en ce qu'on utilise, comme alcoolate de lithium, un sel lithié dérivé d'anions chiraux choisi parmi les sels de lithium des composés azotés hydroxylés de formule (III), telle que définie à la revendication 4, dans laquelle Z sert à définir un groupe OH.

13. Procédé selon la revendication 12 caractérisé en ce qu'on utilise le (1R,2S)-2-(N-méthyl-N-isopropylamino)-1-phénylpropanolate de lithium.

14. Utilisation de (-)-(1'S)-1-(2′,2′,6′-triméthyl-2′-cyclohexène-1′-yl)-2-butène-1-one à titre d'ingrédient parfumant ou aromatisant.

15. Utilisation selon la revendication 14 caractérisée en ce que la (-)-(1'S)-1-(2′,2′,6′,-triméthyl-2′-cyclohexène-1-yl)-2-butène-1-one est accompagnée par des quantités mineures de (+)-(1'R)-1-(2′,2′,6′-triméthyl-2′-cyclohexène-1′-yl)-2-butène-1-one.

## Claims

1. (-)-(1'S)-1-(2',2',6'-Trimethyl-2'-cyclohexen-1'-yl)-2-buten-1-one in substantially pure form.

2. Compound according to claim 1, characterized by an [α]²⁰_{D} = -488° (CHCl₃; c = 4.0).

3. (-)-(1'S)-1-(2',2',6'-Trimethyl-2'-cyclohexen-1'-yl)-3-buten-1-one.

4. Process for the preparation of (-)-(1'S)-1-(2',2',6'-trimethyl-2'-cyclohexen-1'-yl)-2-buten-1-one of formula 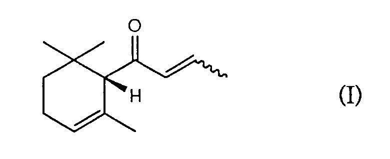 wherein the wavy line designates a C-C bond of cis or trans configuration, characterized in that an enolate of formula 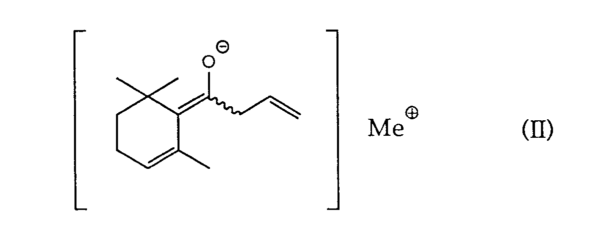 wherein the wavy line has the meaning given above and Me designates an alkali metal atom, preferably lithium or magnesium, is treated with a proton donating chiral reagent consisting of a bifunctional nitrogen derivative of formula 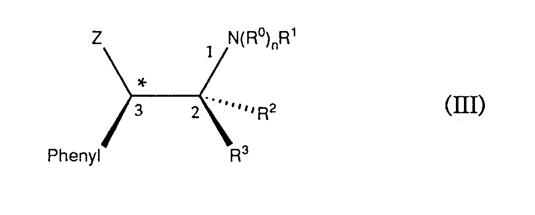 wherein
· the asterisk defines a center of chirality ;
· index n stands for zero or 1 ;
· each of symbols R⁰ and R¹ defines a linear or branched alkyl or aralkyl radical, or one of them represents a hydrogen atom and the other an alkyl radical as defined above ;
· each of symbols R² and R³ defines a linear or branched alkyl radical, or one of them represents a hydrogen atom and the other an alkyl radical such as defined above ; and
· Z designates an OH group or a divalent radical HN-C(O), the nitrogen atom of which is bound to the carbon atom at position 3 and the carbonyl group is bound to the nitrogen atom at position 1 ;
· and wherein the nitrogen atom at position 1 can be bound to a benzylic group of a polystyrenic resin ;
the reaction mixture is then hydrolized and isomerized by means of an isomerization agent according to current methods.

5. Process according to claim 4, characterized in that there is used, as the proton donating chiral reagent of formula (III), a hydroxy-amine of formula 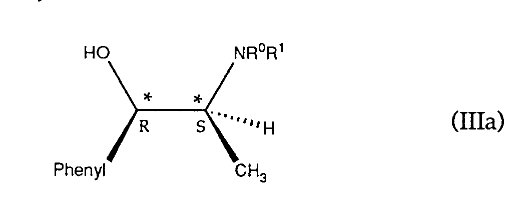 wherein R⁰ and R¹ have the meaning given in claim 4.

6. Process according to claim 5, characterized in that there is used, as the proton donating chiral reagent, (1R,2S)-2-(isopropylamino)-1-phenyl-propan-1-ol, (1R,2S)-2-(N-methyl-N-isopropylamino)-1-phenylpropan-1-ol or (1R,2S)-2-(methylamino)-1-phenylpropan-1-ol.

7. Process according to claim 5, characterized in that one uses as the proton donating chiral reagent a modified polystyrenic resin containing (1R,2S)-2-(methylamino)-1-phenylpropan-1-ol units.

8. Process according to claim 4, characterized in that there is used, as the proton donating chiral reagent of formula (III), a cyclic derivative of urea of formula 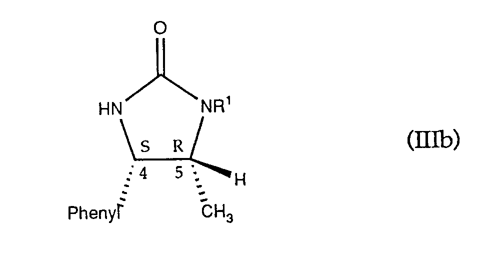 wherein R¹ has the meaning given in claim 4.

9. Process according to claim 8, characterized in that, as the proton donating chiral reagent, (+)-(4S,5R)-1,5-dimethyl-4-phenyl-2-imidazolidone is used.

10. Process for the preparation of (-)-(1'S)-1-(2',2',6'-trimethyl-2'-cyclohexen-1'-yl)-2-buten-1-one of formula 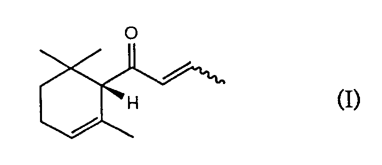 wherein the wavy line designates a C-C bond of cis or trans configuration, characterized in that :
a. an organo-magnesium compound of formula 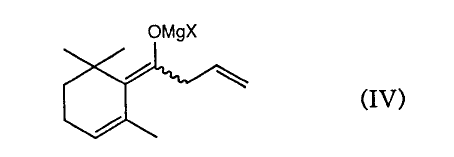 wherein the wavy line has the meaning indicated above and X designates a halogen atom, is treated with at least one equivalent of a lithium alkoxide ;
b. one equivalent at least of a proton donating chiral reagent consisting of a bifunctional nitrogen derivative of formula (III) as defined in claim 4 is added to the reaction mixture;
c. the reaction mixture is then hydrolized and isomerized by means of an isomerizing agent according to current methods ;
or
a'. an organo-magnesium compound of formula (IV) is treated with one equivalent at least of a lithium alkoxide consisting of a lithium salt of a hydroxylic nitrogen compound of formula (III) as defined in claim 4 ;
b'. one equivalent at least of a proton donor consisting of an aliphatic alcohol, in particular tert-butanol, is added to the reaction mixture ; and
c'. one hydrolizes and isomerizes as indicated under letter c. above.

11. Process according to claim 10, characterized in that, as the lithium alkoxide, a lithium salt derivative of a lower aliphatic alcohol chosen amongst the group consisting of methanol, ethanol, isopropanol and butanol, preferably tert-butanol, is used.

12. Process according to claim 10, characterized in that, as the lithium alkoxide, a lithium salt derivative of chiral anions, chosen amongst the lithium salts of a hydroxylic nitrogen compounds of formula (III) as defined in claim 4, wherein Z stands for an OH group, is used.

13. Process according to claim 12, characterized in that one uses lithium (1R,2S)-2-(N-methyl-N-isopropylamino)-1-phenylpropoxide.

14. Use of (-)-(1'S)-1-(2',2',6'-trimethyl-2'-cyclohexen-1'-yl)-2-buten-1-one as perfuming or flavouring ingredient.

15. Use according to claim 14, characterized in that (-)-(1'S)-1-(2',2',6'-trimethyl-2'-cyclohexen-1'-yl)-2-buten-1-one is accompanied by minor amounts of (+)-(1'R)-1-(2',2',6'-trimethyl-2'-cyclohexen-1'-yl)-2-buten-1-one.

## Patentansprüche

1. (-)-(1'S)-1-(2',2',6'-Trimethyl-2'-cyclohexen-1'-yl)-2-buten-1-on in praktisch reiner Form.

2. Verbindung gemäß Anspruch 1, durch einen [α]²⁰_{D} = -488° (CHCl₃ ; c = 4.0) gekennzeichnet.

3. (-)-(1'S)-1-(2',2',6'-Trimethyl-2'-cyclohexen-1'-yl)-3-buten-1-on.

4. Verfahren zur Herstellung von (-)-(1'S)-1-(2',2',6'-Trimethyl-2'-cyclohexen-1'-yl)-2-buten-1-on der Formel 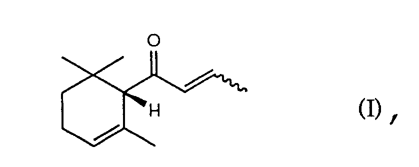 worin die wellige Linie eine cis oder trans C-C Bindung darstellt, dadurch gekennzeichnet daß man ein Enolat der Formel 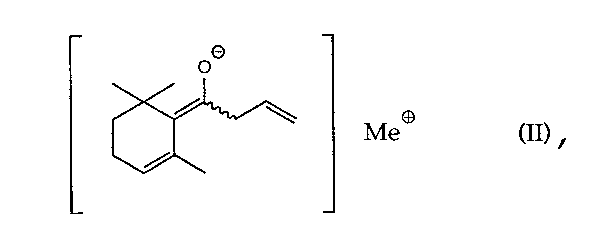 in welcher die wellige Linie die angegebene Bedeutung hat, und Me ein Alkalimetallatom, vorzugsweise Lithium oder Magnesium, definiert, mit einem chiralen Protonendonator-reagens, das aus einem bifunktionalen Stickstoffderivat der Formel 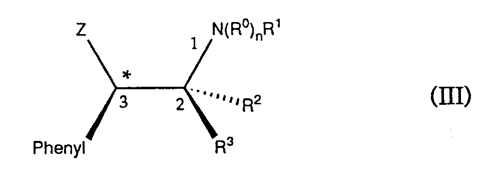 besteht, worin
· das Sternchen ein Chiralitätszentrum definiert ;
· der Index n null oder 1 ist ;
· jedes der Symbolen R⁰ und R¹ einen lineare oder verzweigte Alkyl- oder Aralkylrest definiert, oder eines der beiden ein Wasserstoffatom und das andere einen wie oben definierten Alkylrest bedeutet ;
· jedes der R² und R³ einen lineare oder verzweigte Alkylrest bedeutet, oder eines der beiden ein Wasserstoffatom und das andere einen wie oben definierten Alkylrest bedeutet; und
· Z für eine OH Gruppe oder für einen bivalenten HN-C(O) Rest steht, dessen Stickstoffatom am Kohlenstoffatom 3 und dessen Carbonylgruppe am Kohlenstoffatom 1 gebunden ist ;
· und worin das Stickstoffatom in der Stellung 1 an eine Benzylgruppe eines Polystyrolharzes gebunden sein kann ;
reagiert, und dann das Reaktionsgemisch hydrolysiert, und mittels eines Isomerisierungsmittels nach üblichen Methoden isomerisiert.

5. Verfahren gemäß Anspruch 4, dadurch gekennzeichnet, daß man als chirales Protonendonator-reagens der Formel (III) ein Hydroxyamin der Formel 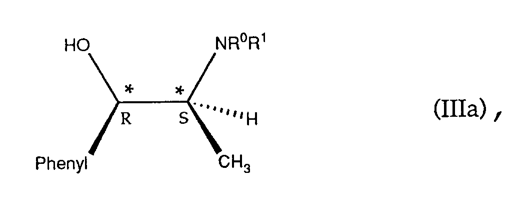 worin R⁰ und R¹ die im Anspruch 4 angegebene Bedeutung haben, verwendet.

6. Verfahren gemäß Anspruch 5, dadurch gekennzeichnet, daß man als chirales Protonendonator-reagens (1R,2S)-2-(Isopropylamino)-1-phenylpropan-1-ol, (1R,2S)-2-(N-Methyl-N-isopropylamino)-1-phenyl-propan-1-ol oder (1R,2S)-2-(Methylamino)-1-phenylpropan-1-ol verwendet.

7. Verfahren gemäß Anspruch 5, dadurch gekennzeichnet, daß man als chirales Protonendonator-reagens ein modifiziertes Polystyrolharz, welches (1R,2S)-2-(Methylamino)-1-phenylpropan-1-ol Einheiten enthält, verwendet.

8. Verfahren gemäß Anspruch 4, dadurch gekennzeichnet, daß man als chirales Protonendonator-reagens der Formel (III) ein Ringderivat des Harnstoffs der Formel 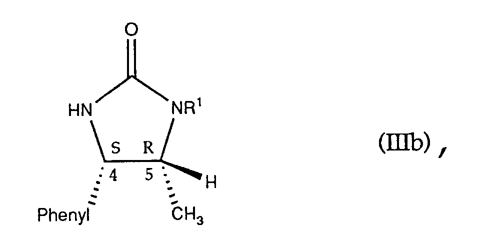 worin R¹ die im Anspruch 4 angebene Bedeutung hat, verwendet.

9. Verfahren gemäß Anspruch 8, dadurch gekennzeichnet, daß man als chirales Protonendonator-reagens (+)-(4S,5R)-1,5-Dimethyl-4-phenyl-2-imidazolidon verwendet.

10. Verfahren zur Herstellung von (-)-(1'S)-1-(2',2',6'-Trimethyl-2'-cyclohexen-1'-yl)-2-buten-1-on der Formel 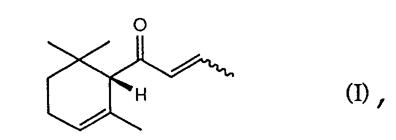 worin die wellige Linie eine cis oder trans C-C Bindung darstellt, dadurch gekennzeichnet, daß man
a. eine Organomagnesiumverbindung der Formel 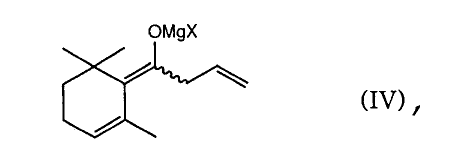 worin die wellige Linie die obengenannte Bedeutung hat, und X ein Halogenatom darstellt, mit wenigstens einem Äquivalent eines Lithiumalkoholats behandelt;
b. an das Reaktionsgemisch wenigstens ein Äquivalent eines chiralen Protonendonator-reagens, das aus einem bifunktionalen Stickstoffderivat der Formel (III) nach Anspruch 4 besteht, addiert;
c. dann das Reaktionsgemisch hydrolisiert, und mittels eines Isomerisierungsmittels nach üblichen Methoden isomerisiert;
oder, daß man
a'. eine Organomagnesiumverbindung der Formel (IV) mit wenigstens einem Äquivalent eines Lithiumalkoholat, das aus einem Lithiumsalz einer hydroxylierten Stickstoffverbindung der Formel (III) nach Anspruch 4 besteht, behandelt ;
b'. an das Reaktionsgemisch wenigstens ein Äquivalent eines chiralen Protonendonator-reagens addiert, welches aus einem aliphatischen Alkohol, besonders tert-Butanol besteht; und
c'. wie unter c. hydrolisiert und isomerisiert.

11. Verfahren gemäß Anspruch 10, dadurch gekennzeichnet, daß man als Lithiumalkoholat ein Derivat eines niedrigen Alkohols verwendet, welcher unter Methanol, Ethanol, Isopropanol und Butanol, vorzugsweise tert-Butanol ausgewählt wird.

12. Verfahren gemäß Anspruch 10, dadurch gekennzeichnet, daß man als Lithiumalkoholat ein von chiralen Anionen abgeleitetes Lithiumsalz verwendet, welches unter den Lithiumsalzen der hydroxylierten Stickstoffverbindungen der Formel (III) nach Anspruch 4, worin Z eine OH Gruppe darstellt, ausgewählt wird.

13. Verfahren gemäß Anspruch 12, dadurch gekennzeichnet, daß man Lithium (1R,2S)-2-(N-Methyl-N-isopropylamino)-1-phenylpropanolat verwendet.

14. Verwendung von (-)-(1'S)-1-(2',2',6'-Trimethyl-2'-cyclohexen-1'-yl)-2-buten-1-on als Riechstoff oder Geschmackstoff.

15. Verwendung gemäß Anspruch 14, dadurch gekennzeichnet, daß (-)-(1'S)-1-(2',2',6'-Trimethyl-2'-cyclohexen-1'-yl)-2-buten-1-one, von kleineren Menge (+)-(1'R)-1-(2',2',6'-Trimethyl-2'-cyclohexen-1'-yl)-2-buten-1-ons begleitet ist.
